(19) Europäisches Patentamt
European Patent Office
Office européen des brevets

(11) Publication number: **0 619 074 A1**

(12) # EUROPEAN PATENT APPLICATION

(21) Application number: **94105069.2**

(22) Date of filing: **30.03.94**

(51) Int. Cl.5: **A01N 57/12**, A01N 37/10, A01N 37/06, A61K 7/48, //(A01N57/12,37:10,37:06)

(30) Priority: **30.03.93 US 40069**

(43) Date of publication of application: **12.10.94 Bulletin 94/41**

(84) Designated Contracting States: **BE DE ES FR GB IT NL SE**

(71) Applicant: **KIMBERLY-CLARK CORPORATION**
**401 North Lake Street**
**Neenah, Wisconsin 54956-0349 (US)**

(72) Inventor: **Touchet, Yvette Lynn**
**208 North Street**
**North Fond du Lac, Wisconsin 54937 (US)**
Inventor: **Radovanovich, Peter Michael**
**725 Oak Street**
**Neenah, Wisconsin 54956 (US)**

(74) Representative: **Diehl, Hermann, Dr. Dipl.-Phys. et al**
**DIEHL, GLÄSER, HILTL & PARTNER**
**Patentanwälte**
**Flüggenstrasse 13**
**D-80639 München (DE)**

(54) **Antimicrobially effective aqueous solution and preserved wet wipes using same.**

(57) An antimicrobially effective solution especially suitable for use in baby wipes, can be antimicrobially effective against bacteria, yeasts and molds if the solution contains a phospholipid and sufficient sorbic acid and/or benzoic acid to provide a pH from about 3.5 to about 7. Wet wipes preserved with such a solution are also disclosed.

EP 0 619 074 A1

Wet wipes of various types are well known products useful for a variety of purposes. The solutions incorporated into these products usually contain a number of ingredients intended to enhance or impart particular properties to the product. These properties may relate to, for example, cleaning efficacy, fragrance, medication, reduced irritation, and the like.

For baby wipes in particular, a solution providing a gentle soothing feel while maintaining bactericidal efficacy is highly desireable for product performance. To date, achieving such product performance has been approached with the use of what could be classified as synthetic ingredients, as opposed to natural ingredients. While natural or naturally-based ingredients have been used in the personal care industry for many years because of their perceived ability to enhance skin health, an inherent difficulty with natural ingredients is that they are often unstable and prone to degradation as a function of time and temperature. In addition, natural preservatives fall short of providing the wide spectrum of effectiveness for use as a baby wipe.

Hence there is a need for an antimicrobially effective solution suitable for use in a baby wipe and for a wet wipe having natural or naturally-based ingredients which provides cleansing capability, soothes and protects the skin, and which provides an appropriate antimicrobial activity required for cosmetic products with a minimal number of ingredients.

These objects are solved by the antimicrobially effective solution of any one of independent claims 1 or 4 and the preserved wet wipe of any one of independent claims 9 or 11. Further advantageous features, aspects and details of the invention are evident from the dependent claims, the description and the examples.

The claims are intended to be understood as a first non-limiting approach of defining the invention in general terms.

The invention is based on the findings that a wet wipe can be provided with an improved soothing feel using naturally-derived ingredients with adequate antimicrobial effectiveness. The invention according to a specific aspect resides in a wet wipe comprising a sheet substrate containing a solution, said solution comprising effective amounts of a phospholipid and one or more preservatives selected from the group consisting of sorbic acid, benzoic acid, salts of sorbic acid, and salts of benzoic acid, said solution having a pH of from about 3.5 to about 7, preferably from about 4 to about 6, and being antimicrobially effective against certain bacteria, yeasts and molds as hereinafter defined. Deposition of the phospholipid onto the skin provides a soothing feel, while the combination of the phospholipid and the sorbic acid provide wide spectrum antimicrobial effectiveness. Such wet wipes are particularly useful as baby wipes.

In another aspect, the invention resides in a preserved solution comprising antimicrobially effective amounts of a phospholipid and sorbic acid and/or one or more salts of sorbic acid.

The wet wipe substrate can be any sheet or wipe which retains its integrity while carrying the solution. Suitable substrates include, without limitation, nonwoven sheets such as thermoplastic meltblown sheets, bonded carded webs, airlaid sheets, and the like. A nonwoven coform meltblown sheet containing polypropylene fibers and pulp fibers, as described in U.S. Patent No. 4,100,324 to Anderson et al. issued July 11, 1978 and entitled "Nonwoven Fabric and Method of Producing Same", which is herein incorporated by reference, is preferred for wet wipes to be used as baby wipes because of its softness. Preferable basis weights for such a sheet substrate can be from about 35 to about 100 grams per square meter, more preferably from about 70 to about 80 grams per square meter.

The amount of solution carried by the sheet substrate can preferably be from about 300 to about 500 weight percent based on the weight of the sheet substrate, more preferably from about 350 to about 450 weight percent.

Suitable phospholipids are triglyceride-like fatty acids substituted by quaternary amine compounds and having the following structural formula:

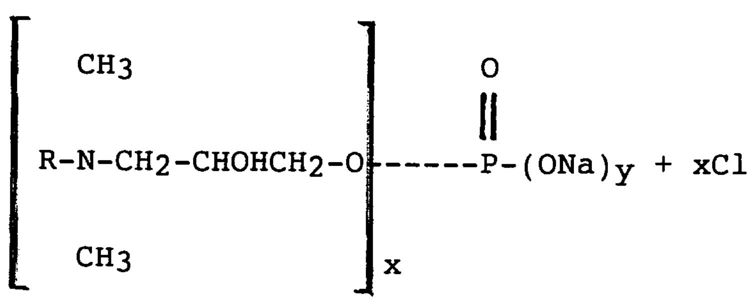

wherein R is an aliphatic oil group having a carbon chain of from about 6 to about 30 carbon atoms, suitably from about 16 to about 26 carbon atoms; and wherein x + y = 3. Preferred R groups are cocamidopropyl (carbon chain length of 18) or linoleamidopropyl (carbon chain length of 26). In general, carbon chain lengths greater than 30 have less preservative efficacy. A preferred phospholipid of the cocamidopropyl type is available from Mona Industries, Inc., Paterson, New Jersey and identified as Phospholipid PTC. Methods for making such phospholipids are further described in U.S. Patent No. 4,209,449 issued June 24, 1980 to Mayhew et al. entitled "Phosphate Quaternary Compounds" and U.S. Patent No. 4,503,002 issued March 5, 1985 to Mayhew et al. entitled "Phosphate Quaternary Compounds", both of which are herein incorporated by reference.

The amount of phospholipid in the wet wipe solution can be from about 1 to about 3 weight percent, based on the weight of the solution and depending on the particular phospholipid chosen. Amounts below about 1 weight percent become less desireable because the antimicrobial activity of the solution decreases. Amounts greater than about 3 weight percent are less preferred because the wet wipe tends to leave a noticeable phospholipid residue on the user's skin, which is sometimes undesirable for some users. A preferred amount is about 1 weight percent because it is antimicrobially effective and does not leave a noticeable residue.

The sorbic acid component of the solution is particularly unique because it is a natural acid having antifungal activity and which provides the proper pH to synergistically enhance the antimicrobial activity of the phospholipid, as well as buffering the wet wipe solution to approximate the pH of human skin. The amount of sorbic acid or benzoic acid can be any amount sufficient to provide a solution pH of from about 3.5 to about 7, preferably from about 4 to about 6. Suitable amounts of sorbic acid are from about 0.07 to about 0.3 weight percent based on the weight of the solution. More preferred amounts are from about 0.1 to about 0.2 weight percent. At amounts below about 0.07 percent, the sorbic acid is not sufficiently effective against the molds, and the solubility of sorbic acid limits add-on amounts greater than 0.2 percent unless the solution is heated. Similarly, suitable amounts of benzoic acid are from about 0.1 to about 0.3 weight percent based on the weight of the solution.

Preferred salts of sorbic acid and benzoic acid include sodium sorbate, potassium sorbate, sodium benzoate and potassium benzoate. Suitable amounts of the salts can be about 0.2 weight percent or greater, more suitably from about 0.2 to about 1 weight percent.

As used herein, an "antimicrobially effective" wet wipe means that the solution expressed from the wipe has a $D_{10}$-value (the time required for a one-log kill) of 4 hours or less for E. coli (ATCC #25922) and S. aureus (ATCC #6538), both bacteria, and a $D_{10}$-value of 24 hours or less for P. aeruginosa (ATCC #15442), C. albicans (ATCC #10231) (yeasts) and A. niger (ATCC #16404) (mold). Determining $D_{10}$-values involves standard microbiological laboratory practices, but in general terms is determined by inoculating the wet wipe solution with the microbial cultures and recording the decline in the culture colonies over time. The slope of the kill curve is determined by linear regression analysis and the $D_{10}$-value can be calculated by taking the negative reciprocal of the slope. The $D_{10}$-value, which is the time required for a one log kill, can also be calculated as follows:

$$D_{10} = \frac{\text{time}}{\log_{10}N_0 - \log_{10}N_1}$$

where

$N_0$ = initial cell population; and

$N_1$ = survivors at time (t).

Examples

Example 1. Aqueous wet wipe solutions containing different levels of a phospholipid (Phospholipid PTC, Mona Industries, Inc., Paterson, New Jersey) were prepared by adding weighed amounts (1, 2, and 3 weight percent) of the phospholipid to an agitated vessel containing the appropriate amount of water to bring the balance to 100 percent.

The resulting solutions were applied to coform meltblown nonwoven sheets (65 percent pulp / 35 percent polypropylene) at a 350 weight percent add-on level. The basis weight of each sheet was about 75 grams per square meter and each sheet measured about 19 centimeters by 19 centimeters. The solution was applied by immersing each sheet into the solution and thereafter pressing the sheet to remove excess

solution to obtain the target add-on percentage. Eighty wet sheets were stacked in a covered plastic dispensing tub where the sheets were allowed to sit for three days at room temperature to ensure the opportunity for the solution to interact with the pulp/poly sheet.

After three days, between 1/2 and 3/4 of the wipes were removed from the tub and placed in a sterile plastic bag (3500 bag, Seward Medical, London, United Kingdom). The solution was aseptically expressed from the wipes while confined in the bag using a press (Model 44-133, Dake Corporation, Grand Haven, Michigan) and the expressed solution collected in a second stomacher bag. The recovered solution was then tested for antimicrobial efficacy as described herein above. The $D_{10}$-values for solutions tested are set forth in TABLE 1 below.

TABLE 1

| Ingredient | pH | $D_{10}$ Bacteria | $D_{10}$ Yeast | $D_{10}$ Mold |
|---|---|---|---|---|
| 1% Phospholipid | 5.51 | <1 | <3 | * |
| 2% Phospholipid | 5.91 | <1 | <3 | * |
| 3% Phospholipid | 6.03 | <1 | <3 | * |

* Unable to calculate because organism counts exceeded the parameters of the test. (Ineffective).

Example 2. Wet wipes were prepared and tested for microbial efficacy as described in Example 1, except the solution formulations tested contained 1 weight percent aloe vera, 1 weight percent chamomile extract, 1 weight percent Phospholipid PTC, different amounts of citric and malic acid as set forth below, and balance to 100 percent water. The antimicrobial effectiveness of the various combinations of acids is set forth in TABLE 2 below.

TABLE 2

| Ingredients | pH | $D_{10}$ Bacteria | $D_{10}$ Yeast | $D_{10}$ Mold |
|---|---|---|---|---|
| 0.2% citric acid<br>0.3% malic acid | 2.38 | <4 | * | 48.2 |
| 0.3% citric acid<br>0.3% malic acid | 2.36 | <4 | * | 35.2 |
| 0.3% citric acid<br>0.5% malic acid | 2.35 | <4 | * | 54.0 |

* See Example 1.

Example 3. (This Invention) Wet wipes in accordance with this invention were prepared and tested for antimicrobial efficacy as described in Example 1, except the solution formulations consisted of 1.0 weight percent Phospholipid PTC, different levels of sorbic acid as set forth below, and the balance water. The results of the antimicrobial testing are set forth in TABLE 3 below.

TABLE 3

| Ingredients | pH | $D_{10}$ Bacteria | $D_{10}$ Yeast | $D_{10}$ Mold |
|---|---|---|---|---|
| 0.05% sorbic acid | 3.63 | 0.5 | 21.8 | * |
| 0.075% sorbic acid | 3.48 | 1.0 | 24.8 | * |
| 0.1% sorbic acid | 3.41 | 1.0 | 9.2 | 8.4 |

* See Example 1.

The foregoing results illustrate the unique antimicrobial effectiveness of sorbic acid in combination with phospholipids when the sorbic acid level reaches 0.1 weight percent as compared to higher levels of other carboxylic acids.

## Claims

1. An antimicrobially effective aqueous solution having a pH of from 3.5 to 7, preferably from 4 to 6, said solution comprising from 1 to 3 weight percent phospholipid and from 0.1 to 0.3 weight percent of an acid selected from the group consisting of sorbic acid, benzoic acid and mixtures thereof based on the weight of the solution.

2. The antimicrobially effective aqueous solution of claim 1 wherein the acid is sorbic acid in an amount of from 0.1 to 0.2 weight percent.

3. The antimicrobially effective aqueous solution of claim 2 having a pH of about 6.

4. In antimicrobially effective aqueous solution having a pH of from 4 to 6, said solution comprising from 1 to 3 weight percent phospholipid and 0.2 weight percent or greater of a salt selected from the group consisting of salts of sorbic acid, salts of benzoic acid and mixture thereof based on the weight of the solution.

5. The antimicrobially aqueous solution of claim 4 wherein the amount of the salt is from 0.2 to 1 weight percent.

6. The antimicrobially aqueous solution of claims 4 or 5 wherein the salt is selected from the group consisting of sodium sorbate, potassium sorbate, sodium benzoate, potassium benzoate, and mixtures thereof.

7. The antimicrobially aqueous solution of any one of the preceding claims wherein the phospholipid is a cocamidopropyl phospholipid.

8. The antimicrobially aqueous solution of any one of claims 1 to 6 wherein the phospholipid is a linoleamidopropyl phospholipid.

9. A preserved wet wipe being antimicrobially effective and comprising a sheet substrate containing an aqueous solution according to any one of the preceding claims.

10. The wet wipe of Claim 9 wherein the amount of solution is from 300 to 500 weight percent, based on the weight of the sheet substrate.

11. An antimicrobially effective wet wipe comprising a nonwoven sheet substrate and from 300 to 500 weight percent of an aqueous solution, based on the weight of the sheet substrate, said solution having a pH of from 4 to about 6 and comprising from 1 to 3 weight percent phospholipid and from 0.1 to 0.3 weight percent, preferably 0.1 to 0.2 weight percent sorbic acid, based on the weight of the solution.

12. The wet wipe of Claim 11 wherein the phospholipid is a cocamidopropyl phospholipid or a linoleamidopropyl phospholipid.

European Patent Office

# EUROPEAN SEARCH REPORT

Application Number
EP 94 10 5069

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| X<br>Y | US-A-4 816 170 (COLGATE-PALMOLIVE CO.) 28 March 1989<br>* column 3, line 14 - line 44 *<br>* column 2, line 48 - line 54 * | 1<br>2-6,9-11 | A01N57/12<br>A01N37/10<br>A01N37/06<br>A61K7/48<br>//(A01N57/12, 37:10,37:06) |
| X | DATABASE WPI<br>Section Ch, Week 8547,<br>Derwent Publications Ltd., London, GB;<br>Class A96, AN 85-294331<br>& RO-A-86 561 (MIRAJ INTR PROD COSMETIC) 30 April 1985<br>* abstract * | 4-6 | |
| X | PATENT ABSTRACTS OF JAPAN<br>vol. 014, no. 435 (C-0760)18 September 1990<br>& JP-A-02 169 512 (KOBAYASHI KOSE CO LTD) 29 June 1990<br>* abstract * | 1,4-6 | |
| Y | DATABASE WPI<br>Section Ch, Week 9034,<br>Derwent Publications Ltd., London, GB;<br>Class D21, AN 90-258576<br>& JP-A-2 182 999 (YG TAKAOKA TSUSAN) 17 July 1990<br>* abstract * | 1-6,9-11 | **TECHNICAL FIELDS SEARCHED (Int.Cl.5)**<br>A01N<br>A61K |
| Y | EP-A-0 350 275 (SCOTT PAPER COMPANY) 10 January 1990<br>* the whole document * | 1-6,9-11 | |
| D,Y | US-A-4 503 002 (MONA INDUSTRIES INC.) 5 March 1985<br>* column 1, line 18 - column 2, line 6 * | 1,4,7,8,12 | |

-/--

The present search report has been drawn up for all claims

| Place of search | Date of completion of the search | Examiner |
|---|---|---|
| MUNICH | 1 August 1994 | Klaver, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 94 10 5069

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| P,Y | WO-A-93 08807 (FOST, PERELLA & KOMOR) 13 May 1993<br>* page 1, line 31 - page 2, line 30 *<br>* page 4, line 20 - page 5, line 32 *<br>----- | 1-12 | |
| | | | TECHNICAL FIELDS SEARCHED (Int.Cl.5) |

The present search report has been drawn up for all claims

| Place of search | Date of completion of the search | Examiner |
|---|---|---|
| MUNICH | 1 August 1994 | Klaver, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)